# EUROPEAN PATENT APPLICATION

(11) **EP 1 179 589 A1**
(43) Date of publication of application: **13.02.2002**
(21) Application number: 00116253.6
(22) Date of filing: 08.08.2000
(51) Int. Cl.: C12N 15/00, C07K 14/47

(54) **MMX-1, a member of the family of human cancer/testis antigens, a protein encoded thereby and a process for determining whether a tumor sample has metastatic potential**

(71) Applicant: F. HOFFMANN-LA ROCHE AG, 4070 Basel (CH)
(72) Inventor: The designation of the inventor has not yet been filed
(74) Representative: Schreiner, Siegfried, Dr.

(57) **Abstract**

A nucleic acid molecule (MMX-1) with the nucleic acid sequence SEQ ID NO:1 is induced during tumor progression and/or metastasis. The MMX-1 protein of SEQ ID NO:2 is also provided. A process for determining whether a tumor sample has metastatic potential is provided.

## Description

### Background of the Invention

In order for metastasis of cancer to occur, several hurdles must be overcome, such as degradation of the extracellular matrix and basal membrane, intra- and extravasation of vessels of the blood and of the lymphatic system, escape by the attack of the immune system, and homing and colonization of distant organs (Pardee, A.B., Advances in Cancer Res. 65 (1994) 213-227; Ponta, H., et al., Biochem. Biophys. Acta 1198 (1994) 1-10). A further level of complexity is achieved in that different types of cancers make use of different molecular mechanisms for metastasis and exhibit different tropism of metastasis.

Metastasizing and non-metastasizing human melanoma cell lines have been important tools in identifying differentially expressed genes and for investigation of their role in metastasis (Weterman, M.A.J., et al., Cancer Res. 52 (1992) 1291-1296; Weterman, M.A.J., et al., Int. J. Cancer 53 (1993) 278-284; Van Groningen, J.M., et al., Cancer Res. 55 (1995) 6237-6243; Weterman, M.A.J., et al., Int. J. Cancer 60 (1995) 73-81; van Muijen, G.N.P., et al., Int. J. Cancer 48 (1991) 85-91; van Muijen, G.N.P., et al., Clin. Exp. Metastasis 9 (1991) 259-272).

Cell adhesion molecules play an important role in the invasion, dissemination, extravasation and lodging of tumor cells. The interaction of disseminated tumor cells with endothelium and tissue stroma is supposed to be one of the critical steps in tumor progression and metastasis formation (Ebnet, K., et al., Annu. Rev. Immunol. 14 (1996) 155-177; Varner, J.A., and Cheresh, D.A., Curr. Opin. Cell Biol. 8 (1996) 724-730; Albelda, S.M., Lab. Invest. 68 (1993) 4-17).

### Summary of the Invention

In accordance with the present invention, a protein, termed MMX-1 (melanoma metastasis X-linked gene 1), is provided which is upregulated in metastatic cancer cells as compared to their non-metastatic counterparts. MMX-1 may be involved in promotion of several steps of the metastatic cascade. MMX-1 is a specific marker of metastatic cancer cells, due to the fact that it can be presented in an MHC Class I complex to cytotoxic T cells but is not presented naturally because the only non-tumor cells expressing MMX-1 are testis cells which do not present antigens in an MHC Class I context. The MMX-1 gene encodes preferably a polypeptide of SEQ ID NO:2.

The present invention provides a nucleic acid which is upregulated in metastatic tumor cells and which codes for a polypeptide which is induced during tumor progression or metastasis, the nucleic acid being selected from the group consisting of:
(a) SEQ ID NO: 1;
(b) a nucleic acid sequence which hybridizes under stringent conditions with a nucleic acid probe of the complementary sequence of (a);
(c) a nucleic acid sequence which, because of the degeneracy of the genetic code, is not a sequence of (a) or (b), but which codes for a polypeptide having exactly the same amino acid sequence as a polypeptide encoded by a sequence of (a) or (b); and
(d) a nucleic acid sequence which is a fragment of any of the sequences of (a), (b) or (c).

The present invention further provides a purified and isolated polypeptide which has a sequence of SEQ ID NO:2, is encoded by a nucleic acid of SEQ ID NO:1 and is induced during tumor progression or metastasis.

The present invention further provides a process for detecting the presence or absence of at least one specific nucleic acid or mixture of nucleic acids, or distinguishing between two different sequences in said sample, wherein the sample is suspected of containing said sequence or sequences, which process comprises the following steps in order:
(a) incubating said sample under stringent hybridization conditions with a nucleic acid probe which is selected from the group consisting of:
   (i) a nucleic acid sequence of SEQ ID NO:1;
   (ii) a nucleic acid sequence which is complementary to a nucleic acid sequence of (i);
   (iii) a nucleic acid sequence which hybridizes under stringent conditions with the sequence of (i); and
   (iv) a nucleic acid sequence which hybridizes under stringent conditions with the sequence of (ii); and
(b) determining whether said hybridization has occurred.

Moreover, the present invention provides a process for determining whether or not a cancer cell-containing test sample has potential for tumor progression or metastasis, wherein the test sample and a cancer cell-containing sample wherein is free from metastasis are obtained from the same individual or different individuals of the same species, which process comprises the following steps:
(a) incubating each respective sample under stringent hybridization conditions with a nucleic acid probe which is selected from the group consisting of:
   (i) a nucleic acid sequence of SEQ ID NO:1;
   (ii) a nucleic acid sequence which is complementary to a nucleic acid sequence of (i);
   (iii) a nucleic acid sequence which hybridizes under stringent conditions with the sequence of (i); and
   (iv) a nucleic acid sequence which hybridizes under stringent conditions with the sequence of (ii); and
(b) determining the approximate amount of hybridization of each respective sample with said probe, and
(c) comparing the approximate amount of hybridization of the test sample to an approximate amount of hybridization of the sample which is free from metastasis, to identify whether or not the test sample contains a greater amount of the specific nucleic acid or mixture of nucleic acids than does the sample which is free from metastasis.

### Detailed Description of the Invention

The present invention provides the new gene MMX-1, a protein coded thereby, and use of the MMX-1 gene for diagnostics and therapeutics, especially in the field of cancer. In particular, the invention involves the identification of said gene MMX-1 in mammalian, especially in malignant tumor cells. The invention also relates to diagnosis of the metastatic and progression potential of tumor cells.

The invention comprises a nucleic acid molecule (MMX-1) which has upregulated expression in metastatic tumor cells and which is induced during tumor progression and/or metastasis, especially in malignant melanoma and mammary carcinoma cells. The nucleic acid MMX-1 has the sequence SEQ ID NO:1 or it is a nucleic acid which, because of the degeneracy of the genetic code, differs from SEQ ID NO:1, but which encodes preferably the amino acid sequence encoded by the nucleic acid of SEQ ID NO:1.

The invention further comprises a recombinant polypeptide which is coded by the nucleic acid sequences according to the invention, preferably by the DNA sequence shown in SEQ ID NO:1.

The isolated MMX-1 polypeptide can occur in natural allelic variations which differ from individual to individual. Such variations of the amino acids are usually amino acid substitutions. However, they may also be deletions, insertions or additions of amino acids to the total sequence. The MMX-1 protein according to the invention - depending, both in respect of the extent and type, on the cell and cell type in which it is expressed- can be in glycosylated or non-glycosylated form. Polypeptides with metastatic activity can be identified by transfection of MMX-1-negative non-metastasizing tumor cells with expression vectors for MMX-1, establishment of stable transfectants and evaluation of in vitro invasiveness in Matrigel invasion assays and their metastatic capacity after xenografting into nude mice.

"Polypeptide with MMX-1 activity or MMX-1 " means also proteins with minor amino acid variations but with substantially the same MMX-1 activity. Substantially the same means that the activities are of the same biological properties and the polypeptides show (at least 90%, preferably more than 95%) homology, or preferably identity, in amino acid sequence. Homology can be examined by using the BLAST algorithm described by Altschul, S.F., et al., Nucleic Acids Res. 25 (1997) 3389-3402.

The term "nucleic acid molecule or nucleic acid" denotes a polynucleotide molecule which can be, for example, a DNA, RNA, or derivatized active DNA or RNA. DNA and/or RNA molecules are preferred, however.

The term "hybridize under stringent conditions" means that two nucleic acid fragments are capable of hybridization to one another under standard hybridization conditions described in Sambrook et al., Molecular Cloning: A Laboratory Manual (1989) Cold Spring Harbor Laboratory Press, New York, USA. More specifically, "stringent conditions" as used herein refer to hybridization at 65°C in a hybridization buffer consisting of 250 mmol/l sodium phosphate buffer pH 7.2, 7% (w/v) SDS, 1% (w/v) BSA, 1 mmol/l EDTA and 0.1 mg/ml single-stranded salmon sperm DNA. A final wash is performed at 65°C in 125 mmol/l sodium phosphate buffer pH 7.2, 1 mmol/l EDTA and 1% (w/v) SDS.

The phrase "nucleic acid or polypeptide" as used throughout this application refers to a nucleic acid or polypeptide having a MMX-1 activity which is substantially free of cellular material or culture medium when produced by recombinant DNA techniques, or substantially free of chemical precursors or other chemicals when synthesized chemically. Such a nucleic acid is preferably free of sequences which naturally flank the nucleic acid (i.e. sequences located at the 5' and the 3' ends of the nucleic acid) in the organism from which the nucleic acid is derived.

The polypeptides according to the invention can be produced by recombinant means in host cells, using an expression vector, or can be produced synthetically. Non-glycosylated MMX-1 polypeptide is obtained when it is produced recombinantly in prokaryotes. With the aid of the nucleic acid sequences provided by the invention it is possible to search for the MMX-1 gene or its variants in genomes of any desired cells (e.g. apart from human cells, also in cells of other mammals), to identify these and to isolate the desired gene coding for the MMX-1 protein. Such processes and suitable hybridization conditions are known to a person skilled in the art and are described, for example, by Sambrook et al., Molecular Cloning: A Laboratory Manual (1989), Cold Spring Harbor Laboratory Press, New York, USA, and Hames, B.D., Higgins, S.G., Nucleic Acid Hybridisation - A Practical Approach (1985) IRL Press, Oxford, England. In this case the standard protocols described in these publications are usually used for the experiments.

With the aid of such nucleic acids coding for a MMX-1 protein, the protein according to the invention can be obtained in a reproducible manner and in large amounts. For expression in prokaryotic or eukaryotic organisms, such as prokaryotic host cells or eukaryotic host cells, the nucleic acid is integrated into suitable expression vectors, according to methods familiar to a person skilled in the art. Such an expression vector preferably contains a regulatable/inducible promoter. These recombinant vectors are then introduced for the expression into suitable host cells such as, e.g., E. coli as a prokaryotic host cell or Saccharomyces cerevisiae, Teratocarcinoma cell line PA-1 sc 9117 (Büttner et al., Mol. Cell. Biol. 11 (1991) 3573-3583), insect cells, CHO or COS cells as eukaryotic host cells and the transformed or transduced host cells are cultured under conditions which allow expression of the heterologous gene. The isolation of the protein can be carried out according to known methods from the host cell or from the culture supernatant of the host cell. Such methods are described for example by Ausubel I., Frederick M., Current Protocols in Mol. Biol. (1992), John Wiley and Sons, New York. Also in vitro reactivation of the protein may be necessary if it is not found in soluble form in the cell culture.

MMX-1 can be purified after recombinant production by affinity chromatography using known protein purification techniques, including immunoprecipitation, gel filtration, ion exchange chromatography, chromatofocussing, isoelectric focussing, selective precipitation, electrophoresis, or the like.

The invention further comprises recombinant expression vectors which are suitable for the expression of MMX-1, recombinant host cells transfected with such expression vectors, as well as a process for the recombinant production of a protein which is encoded by the MMX-1 gene.

The invention further comprises a method for detecting a nucleic acid molecule of gene MMX-1, comprising incubating a sample (e.g., body fluids such as blood, cell lysates) with the isolated nucleic acid molecule according to the invention and determining hybridization under stringent conditions of said isolated nucleic acid molecule to a target nucleic acid molecule for determination of presence of a nucleic acid molecule which is the MMX-1 gene and therefore a method for the identification of the metastatic potential and/or progression of tumor cells. Quantitative detection can be performed by PCR techniques, preferably by the use of quantitative RT-PCR using, e.g., the LightCycler® of Roche Diagnostics GmbH, DE.

To determine whether a test sample contains tumor cells, the approximate amount of hybridization of the nucleic acid with the target nucleic acid or nucleic acids is determined. The approximate amount of hybridization need not be determined quantitatively, although a quantitative determination is encompassed by the present invention. Typically, the approximate amount of hybridization is determined qualitatively, for example, by a sight inspection upon detecting hybridization. For example, if a gel is used to resolve labelled nucleic acid which hybridizes to target nucleic acid in the sample, the resulting band can be inspected visually. When performing a hybridization of isolated nucleic acid which is free from tumor cells from an individual of the same species, the same protocol is followed. One can compare the approximate amount of hybridization in the test sample to the approximate amount of hybridization in the sample free from tumor cells, to identify whether or not the test sample contains a greater amount of the target nucleic acid or nucleic acids than does the sample which is free from tumor cells.

As is shown in accordance with the present invention, the MMX-1 nucleic acid is present in a greater amount in a metastasized tumor sample than in a sample free from metastasis. A test sample having potential for tumor progression or metastasis will have a greater amount of the MMX-1 nucleic acid of the present invention than does a cancer cell sample which is free from metastasis. To identify a test sample as containing upregulated MMX-1 nucleic acid, i.e., wherein the cancer cells have potential for tumor progression or metastasis, it is preferable that the test sample have an approximate amount of MMX-1 nucleic acid which is appreciably greater that the approximate amount in a non-metastasized sample. For example, a test sample having an upregulated MMX-1 gene may have approximately 15- to approximately 60- fold greater amount of MMX-1 gene than a non-metastasized sample or at least 3-fold greater amount than a housekeeping gene like glyceraldehyde-3-phosphate dehydrogenase (GPDH) or porphobilinogendeaminase (PBGD).

On the basis of the nucleic acids provided by the invention it is possible to provide a test which can be used to detect nucleic acids with upregulated expression in metastatic human tumor cells. Such a test can be carried out by means of nucleic acid diagnostics. In this case the sample to be examined is contacted with a probe that is selected from the group comprising
a) the nucleic acid sequence shown in SEQ ID NO:1 or a nucleic acid sequence which is complementary to said nucleic acid sequence, and
b) nucleic acids which hybridize under stringent conditions with one of the nucleic acids from a), wherein
the nucleic acid probe is incubated with the nucleic acid of the sample and the hybridization is detected optionally by means of a further binding partner for the nucleic acid of the sample and/or the nucleic acid probe. For obtaining a nucleic acid by hybridization in accordance with b), it is preferable to hybridize to the probe shown in SEQ ID NOS:3 to 6 or a sequence complementary thereto. Hybridization between the probe used and nucleic acids from the sample indicates the presence of the RNA of such proteins.

Methods of hybridization of a probe and a nucleic acid are known to a person skilled in the art and are described, for example, in WO 89/06698, EP-A 0 200 362, USP 2915082, EP-A 0 063 879, EP-A 0 173 251, EP-A 0 128 018.

In a preferred embodiment of the invention the coding nucleic acid of the sample is amplified before the test, for example by means of the known PCR technique. Usually a derivatized (labeled) nucleic acid probe is used within the framework of nucleic acid diagnostics. This probe is contacted with a denatured DNA, RNA or RT-DNA from the sample which is bound to a carrier and in this process the temperature, ionic strength, pH and other buffer conditions are selected - depending on the length and composition of the nucleic acid probe and the resulting melting temperature of the expected hybrid - such that the labeled DNA or RNA can bind to homologous DNA or RNA (hybridization see also Wahl, G.M., et al., Proc. Natl. Acad. Sci. USA 76 (1979) 3683-3687). Suitable carriers are membranes or carrier materials based on nitrocellulose (e.g., Schleicher and Schüll, BA 85, Amersham Hybond, C.), strengthened or bound nitrocellulose in powder form or nylon membranes derivatized with various functional groups (e.g., nitro groups) (e.g., Schleicher and Schüll, Nytran; NEN, Gene Screen; Amersham Hybond M.; Pall Biodyne).

Hybridizing DNA or RNA is then detected by incubating the carrier with an antibody or antibody fragment after thorough washing and saturation to prevent unspecific binding. The antibody or the antibody fragment is directed towards the substance incorporated during hybridization to the nucleic acid probe. The antibody is in turn labeled. However, it is also possible to use a directly labeled DNA. After incubation with the antibodies it is washed again in order to only detect specifically bound antibody conjugates. The determination is then carried out according to known methods by means of the label on the antibody or the antibody fragment.

The detection of the expression can be carried out for example as:
- in situ hybridization with fixed whole cells, with fixed tissue smears and isolated metaphase chromosomes,
- colony hybridization (cells) and plaque hybridization (phages and viruses),
- Southern hybridization (DNA detection),
- Northern hybridization (RNA detection),
- serum analysis (e.g., cell type analysis of cells in the serum by slot-blot analysis),
- after amplification (e.g., PCR technique).

Therefore the invention also includes a method for the detection of the metastatic potential of melanoma and mammary carcinoma cells, comprising
a) incubating a sample of body fluid of a patient suffering from cancer, of melanoma cancer cells, of mammary carcinoma cells, or of a cell extract or cell culture supernatants of said cancer cells, whereby said sample contains nucleic acids with a nucleic acid probe which is selected from the group consisting of
   (i) the nucleic acid shown in SEQ ID NO:1 or a nucleic acid which is complementary to said nucleic acid sequence, and
   (ii) nucleic acids which hybridize with the nucleic acids from (i) and
b) detecting hybridization by means of a further binding partner of the nucleic acid of the sample and/or the nucleic acid probe or by X-ray radiography.

In addition, the invention comprises a process for determining whether or not a test sample originating from or containing human cells has a tumor progression potential, which process comprises the following steps:
(a) incubating a first compartiment of said sample under stringent hybridization conditions with a first nucleic acid probe which is selected from the group consisting of:
   (i) a nucleic acid with a sequence of SEQ ID NO:1;
   (ii) a nucleic acid with a sequence which is complementary to the nucleic acid of (i);
   (iii) a nucleic acid with a sequence which hybridizes under stringent conditions with the nucleic acid of (i); and
   (iv) a nucleic acid with a sequence which hybridizes under stringent conditions with the nucleic acid of (ii); and
(b) incubating a second compartiment of said sample under stringent hybridization conditions with a second nucleic acid probe being a housekeeping gene or a fragment thereof;
(c) determining the approximate amount of hybridization of said sample with said first and second probe;
(d) identifying whether or not the test sample contains an at least 3-fold amount of nucleic acid hybridizing with the first probe in comparison to the amount of nucleic acid hybridizing with the second probe.

Preferably the nucleic acid probe is incubated with the nucleic acid of the sample and the hybridization is detected optionally by means of a further binding partner for the nucleic acid of the sample and/or the nucleic acid probe.

The nucleic acids according to the invention are hence valuable prognostic markers in the diagnosis of the metastatic and progression potential of tumor cells of a patient.

The invention further comprises a method for producing a protein whose expression is correlated with tumor metastasis, by expressing an exogenous DNA in prokaryotic or eukaryotic host cells and isolation of the desired protein, wherein the protein is coded by the nucleic acid molecules according to the invention, preferably by the DNA sequence shown in SEQ ID NO:1.

The protein can be isolated from the cells or the culture supernatant and purified by chromatographic means, preferably by ion exchange chromatography, affinity chromatography and/or reverse phase HPLC.

The invention further comprises an isolated protein according to the invention which is encoded by a nucleic acid molecule according to the invention, preferably having the nucleotide sequence set forth in SEQ ID NO:1.

The present invention relates to the cloning and characterization of the gene MMX-1, which is especially characterized as a tumor progression gene, and as an upregulated gene indicative for metastatic potential of melanoma cells. The function of the gene according to the invention (MMX-1) is to promote loss of contact inhibition and anchorage dependence in tumor cells and to promote other essential steps of the metastatic cascade. Therefore the expression of MMX-1 gene correlates with a more aggressive behavior of the tumor cells and also with the potential of the formation of metastasis.

According to the invention inhibitors for the expression of MMX-1 (e.g., antisense nucleotides or antibodies) can be used to inhibit tumor progression/metastasis, preferably of malignant melanomas and mammary carcinomas, in vivo, preferably by somatic gene therapy.

Overall characteristics place the gene in the family of cancer/testis antigens (CTAs) of which the first members, named MAGEs (melanoma antigens), were described by the group of Boon (van der Bruggen et al., Science 254 (1991) 1643-1647).

Regarding the ORF of MMX-1 several features are present which resemble features of other CTAs. The bipartite NLS, though not completely consensus, is also encountered in CTp11 (Zendman, A.J., et al., Cancer Res. 59 (1999) 6223-6229). Bipartite NLS sequences have been described as two basic amino acids (lysine (K) or arginine (R)) separated by a linker of about 10 amino acids from a group of 5 residues of which at least three are basic (Robbins, J., et al., Cell 64 (1991) 615-623). Transfection of MMX-1 cDNA constructs in COS-1 cells showed that its NLS is able to achieve nuclear translocation of the coupled fluorescent protein, resulting in a speckled distribution pattern in which nucleoli are negative. This speckled aspect might be associated with one of the many types of nuclear bodies (Huang, S., et al., J. Cell Biol. 143 (1998) 35-47; Matera, A.G., Trends Cell Biol. 9 (1999) 302-309). Another striking feature is the acidic C-terminus and an overall high amount of charged residues present in the putative MMX-1 protein. As true for the MAGE-type genes highest homology of MMX-1 with other CTAs (especially members of the GAGE family) is shared mainly in the C-terminus (Lucas, S., et al., Cancer Res. 58 (1998) 743-752). Though less consensus compared to for instance CTp11 the C-terminal region of MMX-1 alikes a GAL4 acidic transactivation domain (Mitchell, P.J., and Tjian, R., Science 245 (1989) 371-378). Furthermore CT9 is also implicated in control of gene expression, being a testis-specific member of the RING family of transcriptional regulators (Scanlan, M.J., et al., Cancer Lett. 150 (2000) 155-164). Regarding MMX-1 a role in transcription remains speculative. Since no DNA-binding domain is present, MMX-1 will be dependent on interaction with DNA binding proteins or complexes in order to be able to exert its potential regulatory function.

The expression profile of MMX-1 in normal tissues, tumor cell lines and tumor samples place the gene in the group of cancer/testis antigens, which already include MAGE (Lucas, S., et al., Cancer Res. 58 (1998) 743-752), BAGE (Boel, P., et al., Immunity 2 (1995) 167-175), GAGE (van den Eynde, B., et al., J. Exp. Med. 182 (1995) 689-698), SSX (Gure, A.O., et al., Int. J. Cancer 72 (1997) 965-971), NY-ESO-1 (Chen, Y.T., et al., Proc. Natl. Acad. Sci. USA 94 (1997) 1914-1918), LAGE-1 (Lethe, B., et al., Int. J. Cancer 76 (1998) 903-908), PAGE-1 (Chen, M.E., et al., J. Biol. Chem. 273 (1998) 17618-17625; Brinkmann, U., et al., Proc. Natl. Acad. Sci. USA 95 (1998) 10757-10762) and SCP-1 (Tureci, O., et al., Proc. Natl. Acad. Sci. USA 95 (1998) 5211-5216).

Criteria genes should fullfil to be considered as a member of the family of cancer/testis antigens are formulated in the literature (Chen, Y.T., et al., Proc. Natl. Acad. Sci. USA 94 (1997) 1914-1918; Chen, Y.T., et al., Proc. Natl. Acad. Sci. USA 95 (1998) 6919-6923): (i) predominant expression in testis and generally not in other normal tissues, (ii) induction/activation of mRNA expression in a wide range of human tumors, (iii) expression in malignancies in a lineage-nonspecific fashion, (iv) often existence of multigene families, (v) mapping of the gene, with some exceptions, on the X-chromosome. MMX-1 clearly qualifies as a CTA-family member since it shares criteria i, ii, iii and v.

The MMX-1 gene is localized on the X chromosome (Xp11.21-Xp11.22) between DXS1204 and DXS1199. This position is right next to the cancer/testis families of SSX (Xp11.2) GAGE (Xp11.2-Xp11.4) and MAGE D (Xp11) (de Backer, O., et al., Cancer Res. 59 (1999) 3157-3165; Janssen, B.L., et al., Melanoma Res. 9 (1999) 213-222; de Smet, C., et al., Mol.

Cell Biol. 19 (1999) 7327-7335; Lethe, B., Int. J. Cancer 76 (1998) 903-908; Lucas, S., et al., Int. J. Cancer 87 (2000) 55-60).

With 38% positivity the frequency of MMX-1 expression in melanoma metastases is comparable to SSX-2 (44%), NY-ESO-1 (44%) and MAGE-1 (35%). MAGE-3 (52%) has the highest coverage of mRNA expression in melanoma. Expression of BAGE (22%), GAGE (17%) and SCP-1 (8%) are significantly lower (Sahin, U., et al., Int. J. Cancer 78 (1998) 387-389). The recently described CT10 is present in 50% (n=10) of the melanoma metastases (Gure, A.O., et al., Int. J. Cancer 85 (2000) 726-732), while CT9 is absent in melanoma (Scanlan, M.J., et al., Cancer Lett. 150 (2000) 155-164). For MAGE-3 (76%) (Jungbluth, A.A., et al., Int. J. Cancer 85 (2000) 460-465) and GAGE (41%) (de Backer, O., et al., Cancer Res. 59 (1999) 3157-3165) much higher frequencies melanoma metastases have also been described. These differences in frequency of expression, most probably due to variations in PCR conditions, indicate the difficulty of inter-experiment comparisons. In non-melanoma tumors expression of MMX-1 could only sporadically be detected, whereas several cancer/testis-associated genes are expressed in a wide variety of tumor types.

Expression of CTA genes in testis and cancers is effectuated by a decrease in methylation of CpG islands in their promotor-regions. In placenta hypomethylation has also been described, which might be the reason why some CTAs are sometimes expressed in this type of tissue (de Backer, O., et al., Cancer Res. 59 (1999) 3157-3165). Furthermore, Janssen et al (Janssen, B.L., et al., Melanoma Res. 9 (1999) 213-222) found a correlation between methylation status of tumors and expression of MAGE-type genes. For MAGE-1, demethylation of CpG islands inside the Ets-binding domain was necessary and sufficient to induce expression (de Smet, C., et al., Mol. Cell Biol. 19 (1999) 7327-7335). The LAGE-1 promotor also contains several binding domains for the Ets transcription factor (Lethe, B., Int. J. Cancer 76 (1998) 903-908). Based on the described consensus for Ets binding domains (^{C}/_{A}GGA^{A}/_{T}^{G}/_{A}) (de Smet, C., et al., Immunogenetics 42 (1995) 282-290), one Ets site could be found in the upstream region (-342 of the start codon; AJ400997) of the MMX-1 gene.

The invention further provides methods for identifying and isolation of antagonists of MMX-1 or inhibitors for the expression of MMX-1 (e.g. antisense nucleotides). Such antagonists or inhibitors can be used to inhibit tumor progression or metastasis and cause massive apoptosis of tumor cells in vivo.

According to the invention there are provided methods for identifying and isolation of MMX-1 antagonists which have utility in the treatment of cancer, especially in inhibiting metastasis and related disorders. These methods include methods for modulating the expression of the polypeptides according to the invention, methods for identifying MMX-1 antagonists which can selectively bind to the proteins according to the invention, and methods of identifying MMX-1 antagonists which can modulate the activity of said polypeptides. The methods further include methods for modulating, preferably inhibiting, the transcription of MMX-1 gene to mRNA, which preferably down-regulates the metastatic potential of a tumor cell. These methods can be conducted in vitro or in vivo and may make use of and establish cell lines and transgenic animal models of the invention.

A MMX-1 antagonist is defined as a substance or compound which decreases or inhibits the biological activity of MMX-1, a polypeptide and/or inhibits the transcription or translation of MMX-1 gene. In general, screening procedures for MMX-1 antagonists involve contacting candidate substances with host cells in which invasiveness is mediated by expression of MMX-1 under conditions favorable for measuring MMX-1 activity.

MMX-1 activity may be measured in several ways. Typically, the activation is apparent by a change in cell physiology, such as increased mobility and invasiveness in vitro, or by a change in the differentiation state, or by a change in cell metabolism leading to an increase of proliferation.

The MMX-1 gene and protein of the invention can be used to identify and design drugs which interfere with proliferation and dissemination of tumor cells.

The following examples, references, sequence listing and figures are provided to aid the understanding of the present invention. It is understood that modifications can be made in the procedures set forth without departing from the spirit of the invention.

### Description of the Sequences

- SEQ ID NO:1 :: cDNA and amino acid sequence of MMX-1.
- SEQ ID NO:2 :: Amino acid of MMX-1.
- SEQ ID NO:3 :: Sense primer ß₂ microglobulin.
- SEQ ID NO:4 :: Antisense primer ß₂ microglobulin.
- SEQ ID NO:5 :: Sense primer PBGD.
- SEQ ID NO:6 :: Antisense primer PBGD.
- SEQ ID NO:7 :: Sense primer MMX-1.
- SEQ ID NO:8 :: Antisense primer MMX-1.
- SEQ ID NO:9 :: Sense primer MMX-1 (localized in the first exon).
- SEQ ID NO:10 :: Antisense primer MMX-1 (localized in the first intron).
- SEQ ID NO:11 :: Sense primer for fusion protein.
- SEQ ID NO:12 :: Antisense primer for fusion protein.

### Description of the Figures

- **Figure 1**: MMX-1 cDNA and deduced protein sequence. Primers used for PCR are indicated by arrows. Polyadenylation signal is underlined; putative bipartite nuclear localization signal is boxed; stopcodon is represented by an asterisk. ⊥, marks the exon-intron boundary sites.
- **Figure 2**: A) Northern blot analysis of human melanoma cell lines 1F6 and Mel57. The blot is hybridized with a random-primed cDNA probe from the MMX-1 PCR product (355 bp). B) Ethidium-bromide stained gel showing rRNA bands.
- **Figure 3**: Schematic representation of a 5 kb region containing the human MMX-1 gene and its 463 bp transcription product. The intronic spacing, untranslated regions (UTR), start- (AUG) and stopcodon (UAA), open reading frame (ORF, black box) and nuclear localization signal (NLS, hatched box) are depicted.
- **Figure 4**: A) MMX-1 RT-PCR (355 bp) on RNA from a panel of fresh normal human tissues. B) β₂-Microglobulin control PCR.
- **Figure 5**: RT-PCR on RNA from 4 human melanoma cell lines cultured with and without the demethylating agent 5-aza-2'-deoxycytidine. Names of the specific genes investigated are indicated at the right of the ethidium-bromide stained gel. The bottom panel represents the PBGD control PCR.
- **Figure 6**: Fluorescence microscopy of COS-1 cells transfected with a construct containing ORF for eGFP (A, E, I), MMX-1/eGFP (B, F, J), RFP (C, G, K) or MMX-1/RFP (D, H, L). DAPI images show nuclei (A-D), eGFP fluorescence is shown in (E, F), while (G,H) represents the RFP. Merged images of A and E (I), B and F (J), C and G (K) and D and H (L) are given in the bottom panel.

### Example 1

### Materials and methods

### Human tissues

Lesions from all stages of melanocytic tumor progression (normal skin, common nevi, atypical nevi, primary melanoma and melanoma metastases) and from other tumor specimens were excised from patients at the UMC St. Radboud, Nijmegen, The Netherlands. As normal human tissues there were used disease-free samples from surgically removed tissues or from autopsies with a post-mortem delay shorter than 4 hours. Representative slices from the tissue samples were snap-frozen in liquid nitrogen and stored at -80°C until use.

### Cell lines and primary cell cultures

There were used primary cultures of melanocytes, nevus cells, keratinocytes, dendritic cells, PBMCs, fibroblasts, endothelial cells, pericytes and smooth muscle cells. Human tumor cell lines used were: 14 melanoma cell lines (530, 1F6, MV1, M14, MEL57, BLM, MKR, ZKR, WM164, 451Lu, E10, 518A2, OMM1, OCM1), 7 bladder cell lines (jon, RT4, j82, 5367, 647v, 575a, 253j), 6 colon cell lines (CaCo2, LOVO, Colo320, LS147T, T84, SW1398), 7 prostate cell lines (LNCaP, DU145, PC3, TSU, Alva31, PPC, JCA), 6 kidney cell lines, and 18 cell lines established from other histological tumor types (603, A431, Daudi, H1080, HEL, HepG2, HT29, Jurkat, K562, Kasumi, KM3, LAMA, Molt4, Raji, Ramos, U2OS, U937, Umscc2). Origin and culture conditions for most of the human tumor cell lines and primary cell cultures used, were described by Zendman, A.J., et al., Cancer Res. 59 (1999) 6223-6229 and by Zendman, A.J., et al., FEBS Lett. 446 (1999) 292-298. Colon cell lines Colo320, LS174T and T84 are from ATCC. Culturing of colon carcinoma cell line SW1398 was described by Jansen, W.J., et al., Int. J. Cancer 73 (1997) 891-896.

For demethylation experiments, human melanoma tumor cell lines (530, 1F6, MV1 and BLM) were cultured in the presence of 1 µM of 5-aza-2'-deoxycytidine (DAC; Sigma, Zwijndrecht, The Netherlands) for 72 hours, supplemented to standard medium.

As AML tumor cell lines, cell lines named LAMA and Kasumi are used.

### RNA isolation

Total RNA was isolated from cultured cells using the RNeasy kit (Qiagen, Hilden, Germany). From tissue samples total RNA was isolated by disrupting about 25 frozen sections of 20 µm thickness in 1 ml RNAzolB™ (Campro, Veenendaal, The Netherlands) using a pestle. The RNAzolB™ method was followed by an additional RNeasy cleaning step. For all methods the manufacturer's protocols were followed.

### Suppression subtractive hybridization

Suppression subtractive hybridization (SSH) was performed, comparing 2 pooled common nevi (CN) and 3 pooled melanoma metastases (MM). Nests of melanocytic nevus cells and melanoma cells were isolated by laser-assisted microdissection from multiple frozen sections, stained with toluidin blue for morphological recognition. Locoregional metastases located in the skin to minimize background differences. After total RNA isolation, cDNA synthesis was performed on 100 ng of each RNA sample using the SMART PCR cDNA Synthesis kit (Clontech, Palo Alto, CA). Following successful cDNA amplification, purification and digestion, adapter ligations and subtractive hybridizations were performed with the PCR-Select cDNA Subtraction kit (Clontech). From this, CN-specific cDNAs (CN as tester, and MM as driver), as well as MM-specific cDNAs (MM as tester, and CN as driver) were generated. After two steps of hybridization, two rounds of PCR (30 and 12 cycles) were done to suppress abundantly present differential cDNAs and to increase the total amount of normalized differentially expressed product. Efficiency of subtraction was checked by PCR analysis of the housekeeping gene GAPDH (following manufacturer's protocol) prior to cloning of the end products.

### Cloning and reverse Northern

PCR end products were cloned into a pCR2.1 TOPO-TA cloning vector (Invitrogen, Leek, The Netherlands). From all colonies mini-preps were made. A first redundancy check was performed by determining the length of the different inserts using PCR. Subsequently control hybridization experiments with duplicate nylon membranes were prepared on which equal amounts of insert-specific PCR products (prepared by using nested adapter primers) of all clones were spotted. These blots were hybridized with the subtracted nested PCR products (used for cloning) which were radioactively labeled (additional 20 cycles PCR; [³²P]-dATP). Equal amounts of radioactive-labeled probe were used for hybridization. To select the most differential clones, both the CN-specific and MM-specific probes were hybridized with a blot containing their corresponding clones as well as with a blot of the 'other-way-around' specific clones.

### cDNA sequencing and homology searching

From the selected clones both strands were sequenced using the Dye Terminator Reaction Mix (Perkin Elmer, Norwalk, CT) on an ABI-373 sequencer. Homology searches were performed with BLAST (Altschul, S.F., et al., Nucleic Acids Res. 25 (1997) 3389-3402) and other software on all kinds of public servers of DNA and protein databases of which most were described earlier (Zendman, A.J., FEBS Lett. 446 (1999) 292-298). LabOnWeb (www.labonweb.com) was used to possibly elongate sequences of interest and to get an overview of homologues sequences.

### Northern blotting

Ten micrograms of total RNA were treated and size-separated with the formaldehyde method (Sambrook et al., Molecular Cloning: A Laboratory Manual (1989), Cold Spring Harbor Laboratory Press, New York, USA) on a 1.2% agarose gel and blotted onto a Hybond-N⁺ membrane (Amersham, Aylesbury, UK). The cDNA probe was radiolabeled by [³²P]-dATP incorporation using a random-primed DNA labeling kit (Roche Diagnostics GmbH, Penzberg, Germany). The membrane was hybridized overnight with the radiolabeled probe at 65°C in a hybridization mix (0.25 M sodium phosphate buffer pH 7.2, 7% SDS, 1% BSA, 1 mM EDTA, 0.1 mg/ml single stranded Salmon sperm DNA). Afterwards membranes were washed at 65°C with buffers containing decreasing amounts of salt (1% SDS, 1 mM EDTA and 0.25-0.05 M sodium phosphate pH 7.2), and autoradiographed using Kodak Xomat-S films.

### RT- PCR

Synthesis of cDNA (10' at 25°C; 59' at 42°C; 5' at 95°C) was performed on 1.0 µg of total RNA. The 20 µl reaction mixture contained 250 pmol of random hexadeoxynucleotide primers, 3 mM MgCl₂, 200 µM of each dNTP, 50 mM Tris/HCl pH 8.3, 75 mM KCl, 10 mM DTT and 200 U MMLV reverse transcriptase (Promega, Madison, WI, USA). For amplification one µl of cDNA was supplemented with 2.5 µl PCR-buffer (200 mM (NH₄)₂SO₄, 750 mM Tris/HCl pH 9, 0.1% Tween), 5 µl 1M dNTPs, 10 pmoles of each primer, x µl 25 mM MgCl₂, 0.15 U of Thermoperfectplus™ DNA polymerase (Integro, Zaandam, The Netherlands) and water to a final volume of 25 µl. PCR conditions were 30" at 94°C, 45" at T-annealing (T-ann.) and 90" at 72°C for 30 or 35 cycles. These cycles were preceded by a 3 min. denaturation step at 94°C and followed by a 5 min. elongation step at 72°C. Primer-sequences, cycle numbers, [MgCl₂] and annealing temperatures for each of the cDNA-specific PCRs are given in Table 1. β₂-Microglobulin (β₂M) and/or porphobilinogen deaminase (PBGD) served as control PCRs. DNA molecular weight markers were from Roche Diagnostics GmbH. Per sample 10 µl of product was analyzed on agarose gel using ethidium-bromide staining.

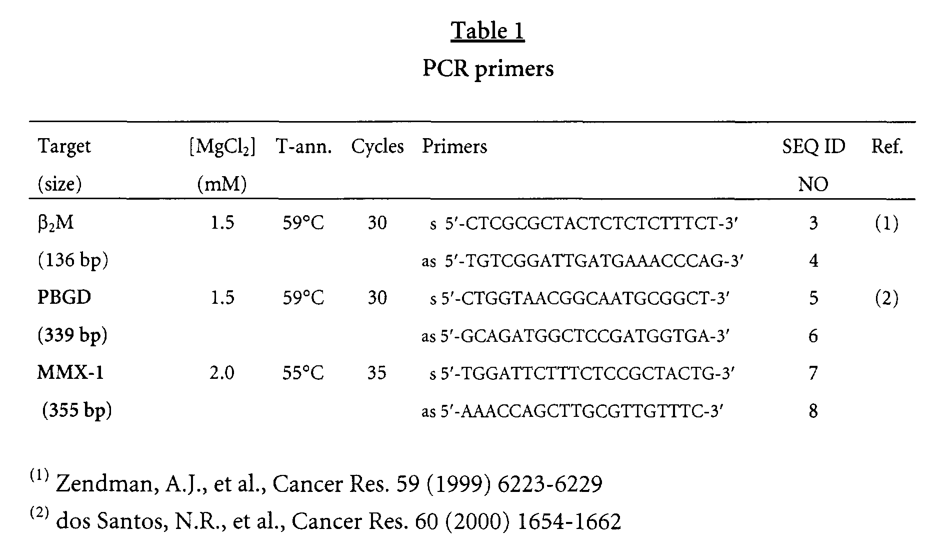

### Chromosomal localization

Genomic localization of the gene was determined by PCR on the Stanford G3RH panel with 83 radiation hybrid clones (Research Genetics Inc., Huntsville, AL) (Stewart, E.A., et al., Genome Res. 7 (1997) 422-433). Primers for this PCR are localized on the first exon (5'-TCCCTGAGGTCTGGATTCTTT-3') (SEQ ID NO:9) and the first intron (5'-CGTCTGGGGAGTCCAGAAT-3') (SEQ ID NO:10) of MMX-1, yielding a DNA-specific product of 337 bp. PCR conditions (30 cycles) were the same as described above for the cDNA PCRs, now with a [MgCl₂] of 1.5 mM and annealing temperature of 60°C.

### Plasmid construction and transfection

Fusion constructs of MMX-1 with eGFP and RFP were constructed for localization studies. Hereto MMX-1 PCR products were generated with primers introducing a BglII-site and mutating the stopcodon (for eGFP-N3: 5'-CCAGATCTAACTTGTGG TTGCTCTTCA-3' (SEQ ID NO:12); for pDsRed-N1: 5'- CCAGATCTCGAACTTGTGGTTGCTCTTCA-3' (SEQ ID NO:6)) in combination with a M13 primer (EcoRI site downstream MMX-1 ORF). The EcoRI and BglII sites were used to fuse the MMX-1 ORF in-frame N-terminally to eGFP (eGFP-N3; EcoRI-BamHI restricted) or RFP (pDsRed-N1; EcoRI-BamHI resticted). This introduced small linkers between the MMX-1 and fluorescent protein ORFs (for MMX1-eGFP: RSIAT; for MMX1-RFP: RDPPVAT). Both vectors coding for the fluorescent proteins were from Clontech, Palo Alto, CA. The in-frame junctions were confirmed by sequencing. Transfections were mediated by FuGENE™6 transfection reagent (Roche Diagnostics GmbH). In short, cells were seeded in 6 well plates and grown till subconfluency. Transfections were performed with 1 µg plasmid construct and 3 µl FuGENE™6 in 2 ml medium. Transient expression of the fusion protein was checked within 48 hours. To visualize expression of the fusion protein, cells (grown on coverslips in 6 well plates) were fixed with 4% paraformaldehyde for 15 minutes at room temperature. Coverslips were put on a glass slide and mounted with 10 µl Tris-buffered glycerol (per 100 ml: 90 ml glycerol; 2 ml Tris/HCl pH 8; 8 ml H₂O) containing 1:4 Vectashield (Vector, Burlingame, CA) and 1:10,000 DAPI (Sigma, Zwijndrecht, The Netherlands). Fluorescent images were obtained using a fluorescence microscope equipped with a CCD camera.

### Example 2

### Cloning, sequencing and homology search

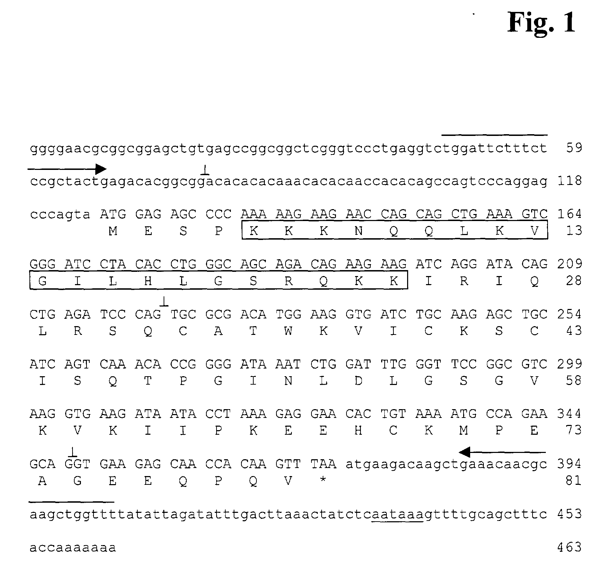

After reverse Northern blotting, control hybridization, and cDNA sequencing, one of the clones specifically expressed in the MM-fraction was selected for further analysis. This 463 bp cDNA (Fig. 1; Genbank Accession No. AJ290447) codes for a putative open reading frame (ORF) of 81 amino acids, harboring a potential bipartite nuclear localization signal (NLS; a.a 5-24). The cDNA also contains a poly-adenylation site. The putative protein is rich in charged residues (27%), especially lysines (15%). With 7 acidic (aspartic acid (D) and glutamic acid (E)) and 15 basic residues (lysine (K) and arginine (R)) the corresponding protein has an overall positive charge. The calculated pI and Mw are 9.65 and 9 kD respectively.

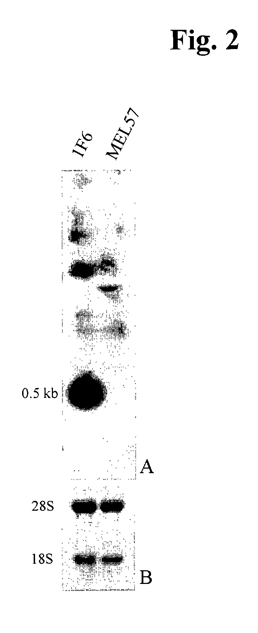

Looking for sequence homologues Genbank and other databases on several servers were inspected. This revealed an Unigene entry (Hs. 112208) of several EST sequences, mainly from testis, with an almost perfect homology to the clone of the invention. To determine whether the cDNA was full-length Northern blotting was performed on RNA from two melanoma cell lines (1F6 and Mel57) and it was also tried to elongate the sequence by electronic 'RACE' with the LabOnWeb program. Northern blotting revealed a band at about 0.5 kb in the 1F6 lane, while Mel57 was negative (Fig. 2A). LabOnWeb was unable to elongate the cDNA. These results strongly indicate the 463 bp cDNA to be full length.

Strongest homology to known human proteins was found with members of the GAGE family of cancer/testis antigens (CTAs), though this comprised of only 33% identity (60% similarity) in the last 30 C-terminal amino acids. On nucleotide-level homology was about 50%.

### Gene structure and chromosomal localization

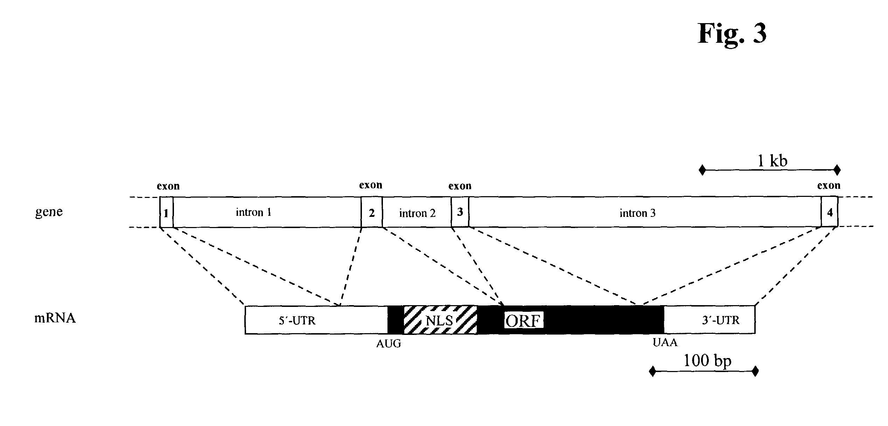

During efforts to PCR and sequence the intronic sequences present in the clone of the invention, a large DNA fragment was found in Genbank (AC025553), containing the full genomic sequence, from which there was deduced the genetic exon-intron build-up (Fig. 3; Genbank Accession No. AJ400997). The gene was spanning about 5 kb, consists of 4 exons and 3 introns (1416, 527 and 2590 bp respectively). The last two introns are situated inside the coding region. All introns have consensus GT/AG boundary sequences (Breatnach, R., and Chambon, P., Annu. Rev. Biochem. 50 (1981) 349-383).

To determine the locus of the gene a DNA-specific PCR on the 83 radiation hybrid clones of the G3RH-panel was performed. This mapped the gene to region Xp11.21-Xp11.22, between the markers DXS1204 and DXS1199. Based on its origin and chromosomal localization the gene was named MMX-1 for Melanoma Metastasis X-linked gene 1.

### Example 3

### Expression profile

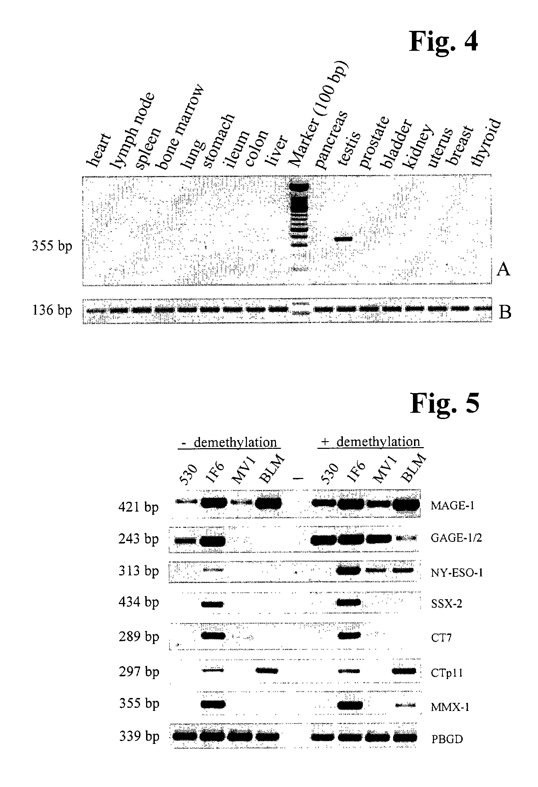

Homology to mainly testis ESTs prompted a looking for expression in a series of normal tissues. Using RT-PCR MMX-1 expression was found mainly in testis, while here all other tissues were negative (Fig. 4A). MMX-1 RT-PCR on a larger number of normal tissues, including other reproductive organs, showed occasional expression in placenta (1 out of 4) and brain (1 out of 6) (Table 2). No expression was detected in 18 other normal tissues, including ovarium, prostate and thyroid.

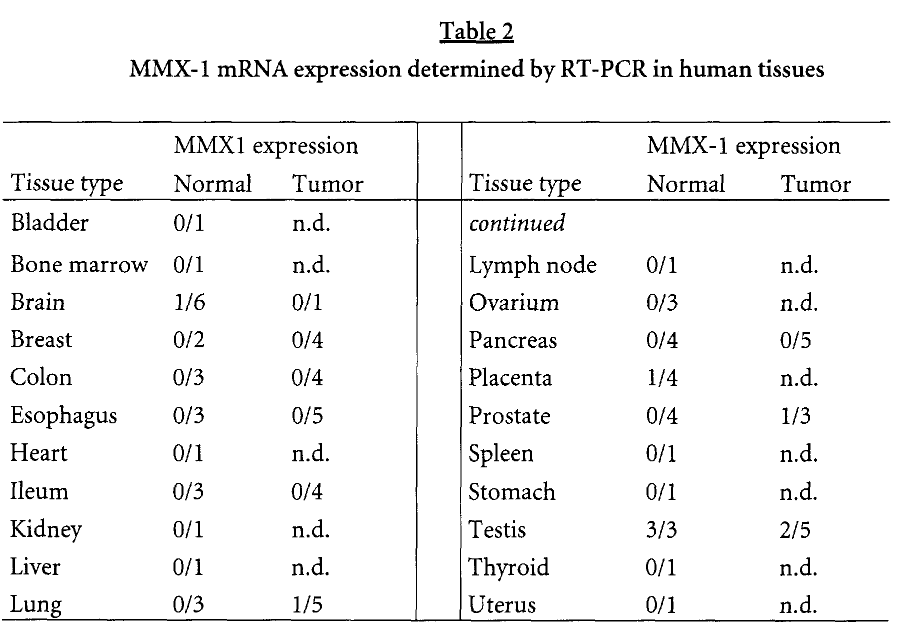

From several tissue types a number of tumors were tested. Regarding non-melanoma samples, expression was occasionally seen: 1 out of 5 lung tumors, 1 out of 3 prostate tumors and 2 out of 5 testicular malignancies-

Expression of MMX-1 in the various stages of human melanocytic tumor progression was confined to melanoma metastases (38%, n=61) (Table 3). No expression was seen in normal skin (n=3), common nevocellular nevus (n=5) and atypical nevus (n=5) and primary cutaneous melanoma (n=8).

**Table 3**

| **MMX-1 mRNA expression determined by RT-PCR in lesions of the different stages of melanocytic tumor progression** | |
|---|---|
| Stage of melanoma lineage | MMX-1 expression |
| Normal skin | 0/3 |
| Common nevocellular nevus | 0/5 |
| Atypical nevus | 0/5 |
| Primary melanoma | 0/8 |
| Melanoma metastasis | 23/61 |

An inventory of MMX-1 expression in primary cell cultures and tumor cell lines showed similar expression patterns compared to fresh tumor material. None of nine different primary cell cultures tested, including melanocytes and nevus cells, were positive. MMX-1 was detected in 43% of the melanoma cell lines (n=14) with 1F6, MKR, ZKR, 518A2, OMM1 and OCM1 being positive. Overall expression in non-melanoma tumor cell lines was limited to 1 (jon) out of 7 bladder cell lines, 1 (Colo320) out of 6 colon cell lines, 2 (LNCaP, DU145) out of 7 prostate cell lines and 2 (Kasumi (AML), U2OS (osteosarcoma)) out of 18 tumor cell lines of other origin (Table 4).

**Table 4**

| **MMX-1 mRNA expression determined by RT-PCR in cultured human tumor cell lines** | |
|---|---|
| Type of cell line | MMX-1 expression |
| Bladder | 1/7 |
| Colon | 1/6 |
| Kidney | 0/6 |
| Melanoma | 6/14 |
| Prostate | 2/7 |
| Other | 2/18 |

Based on expression profile and chromosomal localization, MMX-1 is a cancer/testis-associated gene.

### Example 4

### Upregulation of MMX-1 expression by demethylation

Transcriptional regulation on DNA level by differential methylation of promotors has been described for some CTAs. To see if MMX-1 expression can also be regulated by demethylation, 4 human melanoma cell lines (530, 1F6, MV1 and BLM) were treated with DAC. Induction of expression of some CTAs, known to be responsive to DAC treatment, was done as positive control. As can be seen in Figure 5 strongest effects of the treatment were seen for NY-ESO-1 and GAGE-1/2, with induction in cell lines MV1 and BLM. Furthermore NY-ESO-1 was markedly upregulated in 1F6 cells, as is the case for GAGE-1/2 in 530 cells. MAGE-1 revealed a DAC-dependent upregulation of expression for 530 and MV1. No effects of demethylation were detected for SSX-2, CT7 and CTp11 transcript levels in all cell lines tested. Regarding MMX-1 a weak induction of expression was seen in one (BLM) of the four treated cell lines.

### Example 5

### Cellular localization

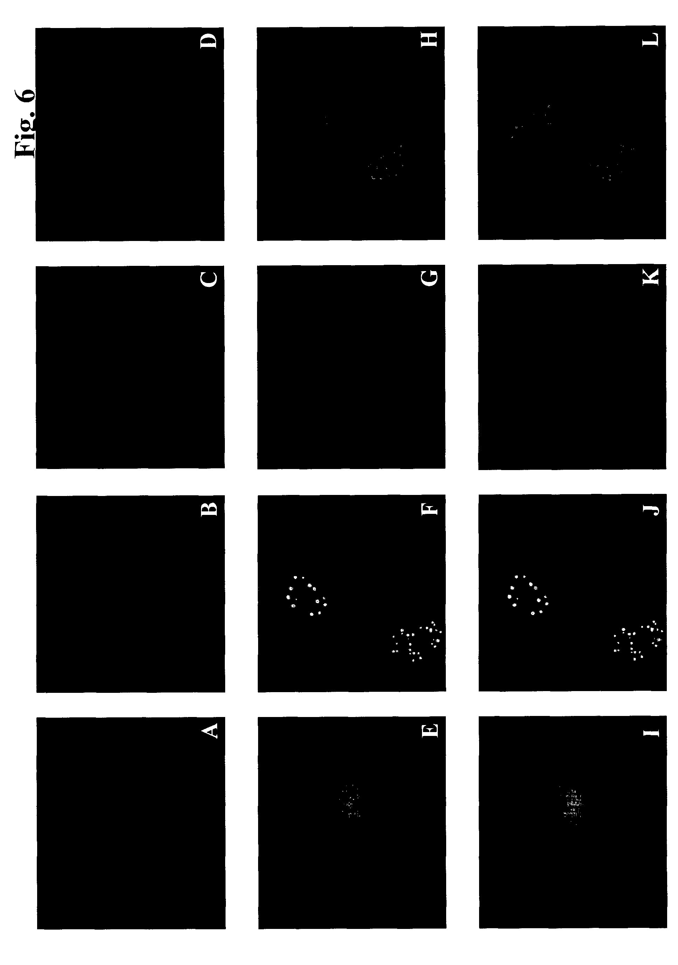

Localization of the MMX-1 protein in the cell was studied by transfecting COS-1 cells with cDNA-constructs of the complete MMX-1 ORF N-terminally fused to the ORF of fluorescent proteins eGFP and RFP. Control transfections with constructs coding for the fluorescent eGFP and RFP only, revealed these proteins to be present in the cytoplasm as well as in the nucleus (Fig. 6E and 6G). Both MMX-1/eGFP and MMX-1/RFP coupled fusion proteins were localized solely in the nucleus, irrespectible of the fluorescent protein used (Fig. 6F and 6H). The fusion protein is visible as distinct intra-nuclear dots, while the nucleoli are negative.

### List of References

Albelda, S.M., Lab. Invest. 68 (1993) 4-17
Altschul, S.F., et al., Nucleic Acids Res. 25 (1997) 3389-3402
Ausubel I., Frederick M., Current Protocols in Mol. Biol. (1992), John Wiley and Sons, New York
Boel, P., et al., Immunity 2 (1995) 167-175
Breatnach, R., and Chambon, P., Annu. Rev. Biochem. 50 (1981) 349-383
Brinkmann, U., et al., Proc. Natl. Acad. Sci. USA 95 (1998) 10757-10762
Büttner et al., Mol. Cell. Biol. 11 (1991) 3573-3583
Chen, M.E., et al., J. Biol. Chem. 273 (1998) 17618-17625
Chen, Y.T., et al., Proc. Natl. Acad. Sci. USA 94 (1997) 1914-1918
Chen, Y.T., et al., Proc. Natl. Acad. Sci. USA 95 (1998) 6919-6923
de Backer, O., et al., Cancer Res. 59 (1999) 3157-3165
de Smet, C., et al., Immunogenetics 42 (1995) 282-290
de Smet, C., et al., Mol. Cell Biol. 19 (1999) 7327-7335
dos Santos, N.R., et al., Cancer Res. 60 (2000) 1654-1662
Ebnet, K., et al., Annu. Rev. Immunol. 14 (1996) 155-177
EP-A 0 063 879
EP-A 0 128 018
EP-A 0 173 251
EP-A 0 200 362
Gure, A.O., et al., Int. J. Cancer 72 (1997) 965-971
Gure, A.O., et al., Int. J. Cancer 85 (2000) 726-732
Hames, B.D., Higgins, S.G., Nucleic Acid Hybridisation - A Practical Approach (1985) IRL Press, Oxford, England
Huang, S., et al., J. Cell Biol. 143 (1998) 35-47
Jansen, W.J., et al., Int. J. Cancer 73 (1997) 891-896
Janssen, B.L., et al., Melanoma Res. 9 (1999) 213-222
Jungbluth, A.A., et al., Int. J. Cancer 85 (2000) 460-465
Lethe, B., et al., Int. J. Cancer 76 (1998) 903-908
Lethe, B., Int. J. Cancer 76 (1998) 903-908
Lucas, S., et al., Cancer Res. 58 (1998) 743-752
Lucas, S., et al., Int. J. Cancer 87 (2000) 55-60
Matera, A.G., Trends Cell Biol. 9 (1999) 302-309
Mitchell, P.J., and Tjian, R., Science 245 (1989) 371-378
Pardee, A.B., Advances in Cancer Res. 65 (1994) 213-227
Ponta, H., et al., Biochem. Biophys. Acta 1198 (1994) 1-10
Sahin, U., et al., Int. J. Cancer 78 (1998) 387-389
Sambrook et al., Molecular Cloning: A Laboratory Manual (1989), Cold Spring Harbor Laboratory Press, New York, USA
Scanlan, M.J., et al., Cancer Lett. 150 (2000) 155-164
Stewart, E.A., et al., Genome Res. 7 (1997) 422-433
Tureci, O., et al., Proc. Natl. Acad. Sci. USA 95 (1998) 5211-5216
USP 2915082
van den Eynde, B., et al., J. Exp. Med. 182 (1995) 689-698
van der Bruggen et al., Science 254 (1991) 1643-1647
van Groningen, J.M., et al., Cancer Res. 55 (1995) 6237-6243
van Muijen, G.N.P., et al., Clin. Exp. Metastasis 9 (1991) 259-272
van Muijen, G.N.P., et al., Int. J. Cancer 48 (1991) 85-91
Varner, J.A., and Cheresh, D.A., Curr. Opin. Cell Biol. 8 (1996) 724-730
Wahl, G.M., et al., Proc. Natl. Acad. Sci. USA 76 (1979) 3683-3687
Weterman, M.A.J., et al., Cancer Res. 52 (1992) 1291-1296
Weterman, M.A.J., et al., Int. J. Cancer 53 (1993) 278-284
Weterman, M.A.J., et al., Int. J. Cancer 60 (1995) 73-81
WO 89/06698
Zendman, A.J., et al., Cancer Res. 59 (1999) 6223-6229
Zendman, A.J., et al., FEBS Lett. 446 (1999) 292-298

<210> 2
<211> 81
<212> PRT
<213> Homo sapiens

<400> 2
Met Glu Ser Pro Lys Lys Lys Asn Gln Gln Leu Lys Val Gly Ile Leu
1               5                   10                  15
His Leu Gly Ser Arg Gln Lys Lys Ile Arg Ile Gln Leu Arg Ser Gln
            20                  25                  30
Cys Ala Thr Trp Lys Val Ile Cys Lys Ser Cys Ile Ser Gln Thr Pro
        35                  40                  45
Gly Ile Asn Leu Asp Leu Gly Ser Gly Val Lys Val Lys Ile Ile Pro
    50                  55                  60
Lys Glu Glu His Cys Lys Met Pro Glu Ala Gly Glu Glu Gln Pro Gln
65                  70                  75                  80
Val

<210> 3
<211> 21
<212> DNA
<213> Artificial Sequence

<220>
<223> Description of Artificial Sequence:sense primer
      ¯2-Microglobulin

<400> 3
ctcgcgctac tctctctttc t                                           21

<210> 4
<211> 21
<212> DNA
<213> Artificial Sequence

<220>
<223> Description of Artificial Sequence:antisense
      primer ¯2-Microglobulin

<400> 4
tgtcggattg atgaaaccca g                                           21

<210> 5
<211> 20
<212> DNA
<213> Artificial Sequence

<220>
<223> Description of Artificial Sequence:sense primer
      PBGD

<400> 5
ctggtaacgg caatgcggct                                             20

<210> 6
<211> 20
<212> DNA
<213> Artificial Sequence

<220>
<223> Description of Artificial Sequence:antisense
      primer PBGD

<400> 6
gcagatggct ccgatggtga                                                20


<210> 7
<211> 21
<212> DNA
<213> Artificial Sequence

<220>
<223> Description of Artificial Sequence:sense primer
      MMX-1

<400> 7
tggattcttt ctccgctact g                                              21


<210> 8
<211> 21
<212> DNA
<213> Artificial Sequence

<220>
<223> Description of Artificial Sequence:antisense
      primer MMX-1

<400> 8
tggattcttt ctccgctact g                                              21


<210> 9
<211> 21
<212> DNA
<213> Artificial Sequence

<220>
<223> Description of Artificial Sequence:sense primer
      MMX-1

<400> 9
tccctgaggt ctggattctt t                                              21


<210> 10
<211> 19
<212> DNA
<213> Artificial Sequence

<220>

<223> Description of Artificial Sequence:antisense
      primer MMX-1

<400> 10
cgtctgggga gtccagaat                                                    19


<210> 11
<211> 27
<212> DNA
<213> Artificial Sequence

<220>
<223> Description of Artificial Sequence:sense primer
      eGFP-N3

<400> 11
ccagatctaa cttgtggttg ctcttca                                           27


<210> 12
<211> 29
<212> DNA
<213> Artificial Sequence

<220>
<223> Description of Artificial Sequence:antisense
      primer pDsRed-N1

<400> 12
ccagatctcg aacttgtggt tgctcttca                                         29

SEQUENCE LISTING

<110> F. HOFFMANN-LA ROCHE AG

<120> MMX-1, a novel member of the family of human
      cancer/testis antigens, a protein encoded thereby, and
      a process for determining whether a tumor sample has
      metastatic potential

<130> Case 20708

<140>
<141>

<160> 12

<170> PatentIn Ver. 2.1

<210> 1
<211> 463
<212> DNA
<213> Homo sapiens

<220>
<221> CDS
<222> (126)..(371)

<400> 1
ggggaacgcg gcggagctgt gagccggcgg ctcgggtccc tgaggtctgg attctttctc 60

cgctactgag acacggcgga cacacacaaa cacacaacca cacagccagt cccaggagcc 120

cagta atg gag agc ccc aaa aag aag aac cag cag ctg aaa gtc ggg atc 170
      Met Glu Ser Pro Lys Lys Lys Asn Gln Gln Leu Lys Val Gly Ile
        1               5                  10                  15

cta cac ctg ggc agc aga cag aag aag atc agg ata cag ctg aga tcc   218
Leu His Leu Gly Ser Arg Gln Lys Lys Ile Arg Ile Gln Leu Arg Ser
                 20                  25                  30

cag tgc gcg aca tgg aag gtg atc tgc aag agc tgc atc agt caa aca   266
Gln Cys Ala Thr Trp Lys Val Ile Cys Lys Ser Cys Ile Ser Gln Thr
             35                  40                  45

ccg ggg ata aat ctg gat ttg ggt tcc ggc gtc aag gtg aag ata ata   314
Pro Gly Ile Asn Leu Asp Leu Gly Ser Gly Val Lys Val Lys Ile Ile
         50                  55                  60

cct aaa gag gaa cac tgt aaa atg cca gaa gca ggt gaa gag caa cca   362
Pro Lys Glu Glu His Cys Lys Met Pro Glu Ala Gly Glu Glu Gln Pro
     65                  70                  75

caa gtt taa atgaagacaa gctgaaacaa cgcaagctgg ttttatatta           411
Gln Val
80

gatatttgac ttaaactatc tcaataaagt tttgcagctt tcaccaaaaa aa         463

## Claims

1. A nucleic acid which is upregulated in metastatic tumor cells and which codes for a polypeptide which induces tumor progression or metastasis, said nucleic acid being selected from the group consisting of:
(a) SEQ ID NO: 1;
(b) a nucleic acid sequence which hybridizes under stringent conditions with a nucleic acid probe of the complementary sequence of (a);
(c) a nucleic acid sequence which, because of the degeneracy of the genetic code, is not a sequence of (a) or (b), but which codes for a polypeptide having exactly the same amino acid sequence as a polypeptide encoded by a sequence of (a) or (b); and
(d) a nucleic acid sequence which is a fragment of any of the sequences of (a), (b) or (c).

2. The nucleic acid according to claim 1, wherein said nucleic acid has a sequence in accordance with SEQ ID NO: 1.

3. An expression vector comprising a nucleic acid of claims 1 to 2.

4. A host transformed by a nucleic acid of claims 1 to 2.

5. A polypeptide which induces tumor progression or metastasis, wherein said polypeptide is encoded by a nucleic acid of SEQ ID NO:1.

6. A process for detecting the presence or absence of at least one specific nucleic acid or mixture of nucleic acids, or distinguishing between two different sequences in said sample, wherein the sample is suspected of containing said sequence or sequences, which process comprises the following steps in order:
(a) incubating said sample under stringent hybridization conditions with a nucleic acid probe which is selected from the group consisting of:
(i) a nucleic acid sequence taken from the group consisting of SEQ ID NO:1;
(ii) a nucleic acid sequence which is complementary to any nucleic acid sequence of (i);
(iii) a nucleic acid sequence which hybridizes under stringent conditions with the sequence of (i); and
(iv) a nucleic acid sequence which hybridizes under stringent conditions with the sequence of(ii); and
(b) determining whether said hybridization has occurred.

7. The process in accordance with claim 6, wherein said nucleic acid probe is bound to a carrier and step (b) comprises determining whether any antibody conjugates are present, and wherein the steps further comprise after step (a) and prior to step (b):
washing the resultant of step (a);
incubating the resultant of the wash step with a labeled antibody against the nucleic acid probe; and
washing the resultant of the incubating step.

8. An isolated nucleic acid which inhibits a nucleic acid in inducing tumor progression and metastasis, said isolated nucleic acid having a sequence selected from the group consisting of:
(a) a nucleic acid sequence which is complementary to SEQ ID NO: 1; and
(b) a nucleic acid sequence which hybridizes under stringent conditions with the sequence of(a).

9. A process for determining whether or not a cancer cell-containing test sample has potential for tumor progression or metastasis, wherein the test sample and a cancer cell-containing sample wherein is free from metastasis are obtained from the same individual or different individuals of the same species, which process comprises the following steps:
(a) incubating each respective sample under stringent hybridization conditions with a nucleic acid probe which is selected from the group consisting of:
(i) a nucleic acid sequence of SEQ ID NO:1;
(ii) a nucleic acid sequence which is complementary to any nucleic acid sequence of(i);
(iii) a nucleic acid sequence which hybridizes under stringent conditions with the sequence of (i); and
(iv) a nucleic acid sequence which hybridizes under stringent conditions with the sequence of (ii); and
(b) determining the approximate amount of hybridization of each respective sample with said probe, and
(c) comparing the approximate amount of hybridization of the test sample to an approximate amount of hybridization of the sample which is free from metastasis, to identify whether or not the test sample contains a greater amount of the specific nucleic acid or mixture of nucleic acids than does the sample which is free from metastasis.

10. A process for determining whether or not a test sample originating from or containing human cells has a tumor progression potential, which process comprises the following steps:
(a) incubating a first compartiment of said sample under stringent hybridization conditions with a first nucleic acid probe which is selected from the group consisting of:
(i) a nucleic acid with a sequence of SEQ ID NO:1;
(ii) a nucleic acid with a sequence which is complementary to any nucleic acid of (i);
(iii) a nucleic acid with a sequence which hybridizes under stringent conditions with the nucleic acid of (i); and
(iv) a nucleic acid with a sequence which hybridizes under stringent conditions with the nucleic acid of(ii); and
(b) incubating a second compartiment of said sample under stringent hybridization conditions with a second nucleic acid probe being a housekeeping gene or a fragment thereof;
(c) determining the approximate amount of hybridization of said sample with said first and second probe;
(d) identifying whether or not the test sample contains an at least 3-fold amount of nucleic acid hybridizing with the first probe in comparison to the amount of nucleic acid hybridizing with the second probe.
